# EUROPÄISCHE PATENTANMELDUNG

(11) **EP 3 798 202 A1**
(43) Veröffentlichungstag der Anmeldung: **31.03.2021**
(21) Anmeldenummer: 19199547.1
(22) Anmeldetag: 25.09.2019
(51) Int. Cl.: C07C 45/75, C07C 67/39, C07C 67/54, C07C 67/58, C07C 47/22, C07C 69/24, C07C 69/54

(54) **VERFAHREN ZUR AUFREINIGUNG VON METHYLMETHACRYLAT VON LEICHTSIEDENDEN KOMPONENTEN**

(71) Anmelder: Röhm GmbH, 64295 Darmstadt (DE)
(72) Erfinder: Die Erfindernennung liegt noch nicht vor
(74) Vertreter: Röhm Patent Association

(57) **Zusammenfassung**

Die vorliegende Erfindung betrifft ein neuartiges Verfahren zur Aufreinigung eines mit leichtsiedenden Komponenten verunreinigten Methylmethacrylates durch Destillation, wobei dieses MMA mittels einer oxidativen Veresterung hergestellt wurde, und als Rohprodukt als leichtsiedende Komponenten Propionsäuremethylester (PRAME), Isobuttersäuremethylester (IBSME) und Methacrolein (MAL) enthält. Das Verfahren ist dabei auf aus C2-basierendem Methacrolein hergestellten MMA, enthaltend die angegebenen leichtsiedenden Komponenten anwendbar. Das Verfahren kann theoretisch aber auch auf aus C4-basierenden Methacrolein hergestellten MMA, welches PRAME und MAL, jedoch keine nennenswerten Mengen an IBSME aufweist, übertragbar sein.

## Beschreibung

### Gebiet der Erfindung

Die vorliegende Erfindung betrifft ein neuartiges Verfahren zur Aufreinigung eines mit leichtsiedenden Komponenten verunreinigten Methylmethacrylates (MMA) durch Destillation, wobei dieses MMA mittels einer oxidativen Veresterung hergestellt wurde, und als Rohprodukt als leichtsiedende Komponenten Propionsäuremethylester (PRAME), Isobuttersäuremethylester (IBSME) und Methacrolein (MAL) enthält. Das Verfahren ist dabei auf aus C2-basierendem Methacrolein hergestellten MMA, enthaltend die angegebenen leichtsiedenden Komponenten anwendbar. Das Verfahren kann theoretisch aber auch auf aus C4-basierenden Methacrolein hergestellten MMA, welches PRAME und MAL, jedoch keine nennenswerten Mengen an IBSME aufweist, übertragbar sein.

### Stand der Technik

Methylmethacrylat (MMA) wird heute mittels diverser Verfahren, die von C₂-, C₃- oder C₄-Bausteinen ausgehen, hergestellt. In einem dieser Verfahren wird MMA durch eine direkte oxidative Veresterungsreaktion von Methacrolein unter Verwendung von Methanol erhalten. Methacrolein wird dabei in der ersten Stufe aus Propanal und Formaldehyd gewonnen. Ein solches Verfahren ist in WO 2014/170223 beschrieben.

Das erfindungsgemäße Verfahren umfasst die Herstellung von Methacrolein, nach dem sogenannten C2-Verfahren aus Formalin und Propionaldehyd in Gegenwart eines sekundären Amins und einer Säure, zumeist einer organischen Säure. Die Umsetzung erfolgt dabei über eine Mannich-ähnliche Reaktion mit anschließender Freisetzung des katalytischen sekundären Amins. Solche Verfahren zur Herstellung von Methacrolein, sind unter anderem in den Druckschriften US 7,141,702, US 4,408,079, JP 3069420, JP 4173757, EP 0317909 und US 2,848,499 beschrieben. Je nach Verfahren können Ausbeuten zwischen 91 und 98 % erzielt werden. In der Regel fällt ein Methacrolein-Strom an, der nach der Reinigung einen Propionaldehyd-Gehalt zwischen 100 ppm und 2 Gew% aufweist. Diese Methacrolein-Qualität ist grundsätzlich für eine nachfolgende Umsetzung gemäß einer direkten oxidativen Veresterung des Methacroleins zu MMA in der Flüssigphase geeignet.

Der Herstellung von MMA aus Methacrolein in der sogenannten direkten oxidativen Veresterung in der Flüssigphase mit Luft und Methanol als Reaktanten und der anschließenden Aufreinigung des Roh-MMA kommt für die vorliegende Erfindung besondere Bedeutung zu.

In den Druckschriften US 5,969,178, US 7,012,039, WO 2014/170223 und WO 2017/046110 werden Verfahren zur Herstellung von MMA mittels oxidativer Veresterung beschrieben. Dabei werden unterschiedliche Prozessführungsoptionen des Zulaufes des Methacroleins in die oxidative Veresterung sowie der Aufbereitung des erzeugten Roh-MMA offengelegt. Für das erfindungsgemäße Verfahren wird dabei die Verfahrensvariante bevorzugt, in der frisches Methacrolein mit dem Reaktoraustrag der oxidativen Veresterung gemischt wird und in einer Fraktionierung derart auftrennt wird, dass im Destillat Methacrolein, Propionaldehyd, weitere Nebenkomponenten, die einen geringeren Siedepunkt als Methacrolein aufweisen, und ein Teil des Methanols enthalten sind. Für das erfindungsgemäße Verfahren sind insbesondere auch andere Verfahrensvarianten denkbar, insbesondere solche, bei denen Propionaldehyd aus der Methacrolein-Synthese im Prozessschritt der oxidativen Veresterung zu Propionsäuremethylester umgesetzt wird. Die Sumpf-Fraktion dieser Fraktion enthält bevorzugt MMA, Wasser, Methacrylsäure, Salze, IBSME, PRAME und weitere organische Schwersieder. Die Verfahrensvariante wurde in WO 2017/046110 beschrieben. Weiterhin wird nach dem erfindungsgemäßen Verfahren in der oxidativen Veresterung aus Propionaldehyd Propionsäuremethylester gebildet. Des Weiteren entsteht Isobuttersäuremethylester. Üblicherweise erfolgt nach einer Extraktion eine Aufbereitung des Roh-MMA mit mehreren Destillationskolonnen. Dieses Roh-MMA enthält beispielsweise Methacrolein, Propionsäuremethylester, Isobuttersäuremethylester, Methanol, Wasser, Methacrylsäure und schwersiedende Nebenkomponenten. Die Aufarbeitungskonzepte gemäß Stand der Technik führen zwar zu einem Methylmethacrylat mit guter Qualität, jedoch zeigen diese erhöhte Spurenmengen der kritischen und schwer abzutrennenden Nebenkomponenten, Propionsäuremethylester und Isobuttersäuremethylester.

US 4,518,462 offenbart ein Verfahren zur Herstellung von MMA, in dem aus einer oxidativen Veresterung von Methacrolein oder einer Veresterung von Methacrylsäure die Abtrennung von Isobuttersäuremethylester (IBSME) aus dem Roh-MMA mit Hexan als Schleppmittel durchgeführt wird. Der Zulauf in die dazugehörige Destillationskolonne enthält dabei Methanol, MMA, Wasser aus der Reaktion und IBSME. Die Destillation wird ohne weitere Zuleitung von Wasser durchgeführt. Als Trennprinzip liegt dieser Destillation zu Grunde, dass das Schleppmittel Hexan das Azeotrop, welches von MMA und Methanol gebildet wird, aufbricht. Dieses Vorgehen zeigt jedoch keinen Effekt auf das Azeotrop, welches IBSME und Methanol bilden. Als Konsequenz wird IBSME im Kopf der Kolonne angereichert, während gereinigtes MMA im Sumpf und Wasser in einem Seitenstrom abgezogen wird. Bei einer anschließend erfolgenden Phasentrennung des Destillats wird Hexan als Hauptbestandteil der organischen Phase, von Methanol und IBSME als Bestandteile einer polaren Phase erhalten. Eine darauffolgende zweite Destillation dieser polaren Phase wird zur Abtrennung des restlichen Hexans und anderer leichtsiedender Komponenten vom Methanol durchgeführt, wobei jedoch IBSME in der Methanol-Phase zurückbleibt. Für eine Recyclierung des Methanols in den Reaktor wäre somit eine dritte, sehr aufwendige Destillation dieses Stroms notwendig. US 4,518,462 gibt eine MMA Rückgewinnung je nach Betriebsbedingungen zwischen 95,4 und 96,2 % an. Dabei beträgt die MMA-Reinheit je nach Fahrweise zwischen 98,36 Gew% und 99,9 Gew.%.

US 4,070,254 beschreibt ein Verfahren zur Herstellung von MMA mittels z.B. einer oxidativen Veresterung eines C4-basierten Methacroleins oder eines anderen Verfahrens. Auch hier ist die Abtrennung von PRAME aus MMA nicht beschrieben. Das Verfahren verzichtet auf den Einsatz eines Schleppmittels. Die destillative Trennung benötigt insgesamt sehr viele Stufen. Dabei basiert das Trennkonzept darauf, dass ein Zulauf, enthaltend MMA und IBSME, sowie optional Wasser zunächst in ein oder zwei Kolonnen mit Dekanter im Destillat destilliert wird. Dabei kann zusätzliches Wasser entweder in den Dekanter oder in den Kolonnenzulauf gegeben werde. Trennprinzip ist die Anreicherung von IBSME in den Köpfen der Kolonnen. Dabei erfolgt eine partielle Abtrennung des IBSME in die jeweilige Wasserphase, was jedes Mal zu einem relevanten MMA-Verlust führt. Wichtiger Stellparameter ist die Wasserzugabe im Verhältnis zum IBSME-Gehalt im Zulauf. Durch eine Optimierung kann der MMA-Verlust reduziert, jedoch nicht vermieden werden. Der organische Strom des Dekanters der zweiten Kolonne fällt als Abfallstrom an oder kann optional in einer Verfahrensvariante in einer dritten Kolonne destilliert werden, wobei MMA als Sumpf der Kolonnen zurückgewonnen wird. Diese Verfahrensvariante ist jedoch - insbesondere in Hinblick auf den Energiebedarf und die benötigte Anzahl von Trennapparaten - sehr aufwendig. Das Verfahren erreicht je nach Fahrweise MMA-Reinheit zwischen 98,99 Gew% und 99,70 Gew.%. Die MMA Rückgewinnung liegt zwischen 95,0 und 98,94 %.

### Aufgaben

Aufgabe der vorliegenden Erfindung war die Bereitstellung eines neuartigen Verfahrens zur Aufarbeitung von aus Methacrolein hergestelltem Alkylmethacrylat, aus dem besonders effizient leichtsiedende Nebenprodukte entfernt werden.

Insbesondere war es die Aufgabe dabei, dies durch eine neuartig in die Aufarbeitung des Alkylmethacrylats integrierte Destillation zu realisieren.

Insbesondere bevorzugt handelt es sich bei dem Alkylmethacrylat um MMA. Dabei war es dann die Aufgabe unter möglichst geringen Energieaufwand aus diesem MMA besonders effizient Propionsäuremethylester PRAME, Isobuttersäuremethylester IBSME, Methacrolein MAL zu entfernen.

Weiterhin war es Aufgabe der vorliegenden Erfindung, dass das neue Verfahren auf die Reinigung von Alkylmethacrylaten, die aus Methacrolein hergestellt wurden, angewendet werden kann, unabhängig davon, ob das Alkylmethacrylat aus dem Methacrolein über eine oxidative Veresterung oder eine Oxidation mit anschließender Veresterung gewonnen wurde. Dabei sollen insbesondere Nebenprodukte entfernt werden, die in einem MMA vorliegen, welches aus Methacrolein aus einer C2-Quelle hergestellt wurde.

Weitere nicht explizite Aufgaben können sich aus den Beispielen, Ansprüchen oder der Beschreibung, wie auch aus dem Gesamtkontext der Erfindung, auch in Hinblick auf den Stand der Technik ergeben.

### Lösung

Gelöst wurden diese Aufgaben mittels einem neuartigen Verfahren zur Herstellung von Alkylmethacrylaten, bei dem in einer ersten Reaktionsstufe in einem Reaktor I Methacrolein hergestellt und dieses in einer zweiten Reaktionsstufe in einem Reaktor II oxidativ mit einem Alkohol in Gegenwart eines sauerstoffhaltigen Gases unter Entstehung eines ungereinigten Alkylmethacrylatstroms verestert wird. Dieses neuartige Verfahren zeichnet sich insbesondere durch folgende Verfahrensaspekte aus:
a. In Reaktor I wird ausgehend von Propionaldehyd und Formalin Methacrolein erhalten. Dabei enthält dieses Methacrolein Propionaldehyd.
b. In Reaktor II und einem optionalen Reaktor III wird darauf dieses im Methacrolein enthaltene Propionaldehyd in einen Propionsäurealkylester umgewandelt. Gleichzeitig wird in mindestens einem dieser Reaktoren ein Isobuttersäurealkylester gebildet.
c. Nach Reaktor II bzw. Reaktor III wird der entstehende Alkylmethacrylat Roh-Strom in einem Aufarbeitungsverfahren zu reinem Alkylmethacrylat aufgearbeitet. Dieses Aufarbeitungsverfahren durchläuft dabei mehrere destillative Trennkolonnen und mindestens eine extraktive Trennung. Der Alkylmethacrylat Roh-Strom enthält dabei anfangs Propionsäurealkylester und Isobuttersäurealkylester.

Erfindungsgemäß weist Verfahrensschritt c mindestens eine Destillationskolonne I, in der Alkylmethacrylat als Sumpf-Fraktion abgetrennt wird, auf. Dabei wird mittels Fraktionierung die Nebenprodukt-Fraktion, enthaltend Methacrolein, sowie Isobuttersäurealkylester und/oder Propionsäurealkylester, als Kopf-Fraktion erhalten.

Es gibt zwei bevorzugte Ausführungsformen für die vorliegende Erfindung. In der ersten dieser Ausführungsformen handelt es sich bei Reaktor II um einen Reaktor, in dem eine oxidative Veresterung des MAL mit einem Alkohol und Sauerstoff zu dem Alkylmethacrylat erfolgt. Diese Reaktion wird bevorzugt in flüssiger Phase durchgeführt und mit Edelmetall-haltigen Katalysatoren katalysiert. Bei dieser Ausführungsform gibt es keinen Reaktor III.

In der zweiten Ausführungsform wird in dem Reaktor II MAL zu Methacrylsäure - bevorzugt in einer Gasphase - oxidiert. Darauf folgt in Reaktor III eine Veresterung dieser Methacrylsäure mit einem Alkohol zu dem Alkylmethacrylat. Diese Stufe in Reaktor III erfolgt bevorzugt in einer Flüssigphase.

Bevorzugt wird in dem erfindungsgemäßen Verfahren aus Verfahrensschritt c ein Alkylmethacrylat mit einem jeweiligen Gehalt an Propionsäurealkylester bzw. Isobuttersäurealkylester von unter 0,1 Gew% erhalten.

In der Regel handelt es sich bei dem im erfindungsgemäßen Verfahren eingesetzten Alkohol um Methanol. Entsprechend handelt es sich bei dem Alkylmethacrylat um MMA, bei dem Isobuttersäurealkylester um Isobuttersäuremethylester und bei dem Propionsäurealkylester um Propionsäuremethylester.

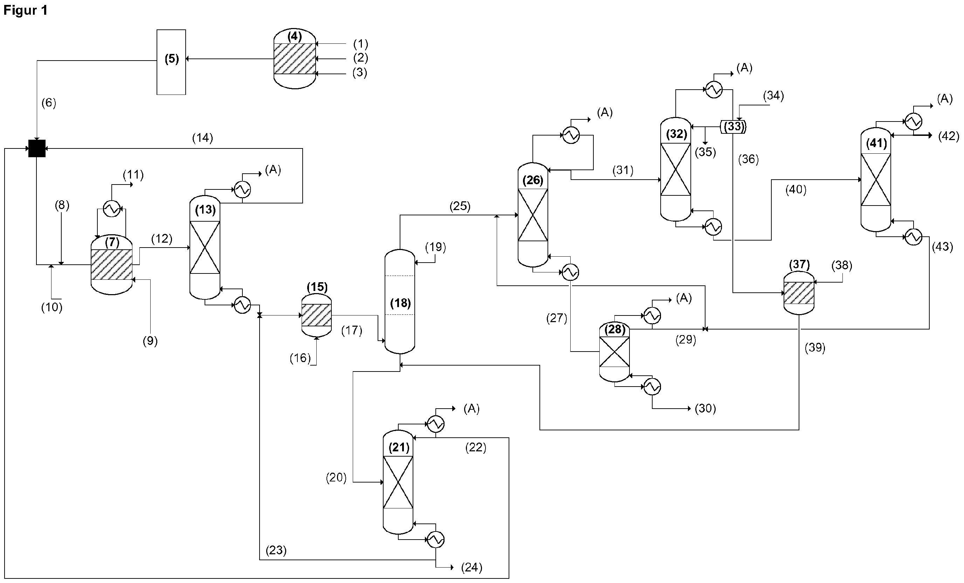

In dieser Variante wird die Kopf-Fraktion aus Destillationskolonne I in einen Phasentrenner I geleitet und dort in eine wässrige und eine organische Fraktion getrennt (siehe Figur 1). Besonders bevorzugt wird dabei zusätzlich Wasser in diesen Phasentrenner I geleitet, wobei es sich bei dem Wasser um Frischwasser und/oder um einen wasserhaltigen Recyclierungsstrom aus einem oder mehreren anderen Prozessschritten handeln kann.

Es ist auch bevorzugt, dass die organische Phase aus Phasentrenner I, die die Nebenprodukte Methacrolein, Isobuttersäurealkylester und Propionsäurealkylester enthält, teilweise oder vollständig in die Destillationskolonne I recycliert wird.

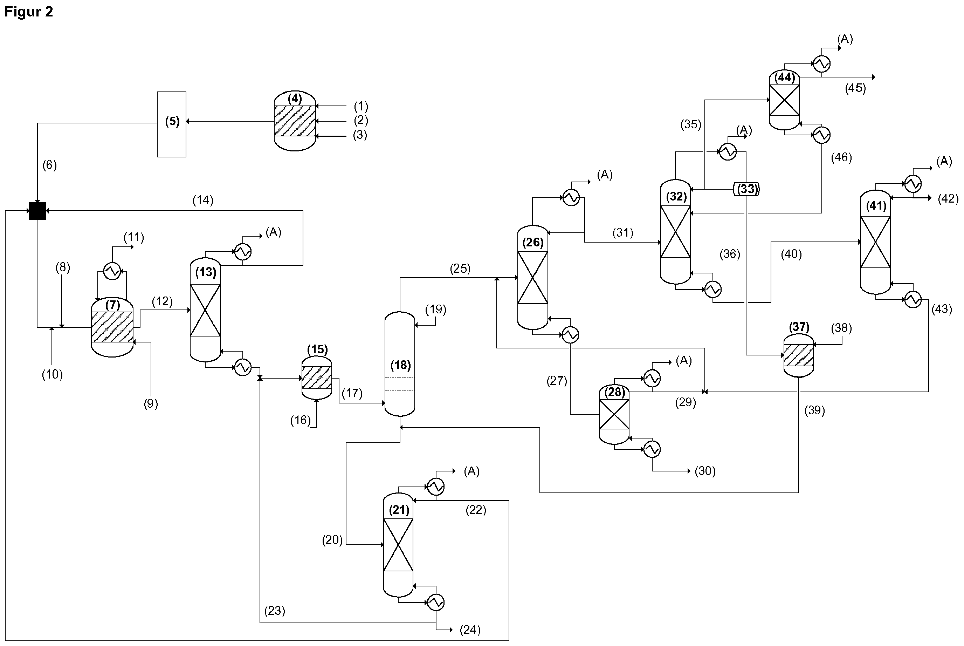

Weiterhin ist es bevorzugt, den Kopf-Strom aus Destillationskolonne I und/oder die organische Phase aus dem Phasentrenner I ganz oder teilweise in eine Destillationskolonne II zur Fraktionierung zu leiten (siehe Figur 2). In dieser Destillationskolonne II erfolgt dann eine Trennung in eine leichtsiedende Nebenprodukt-Fraktion, enthaltend Methacrolein, sowie Isobuttersäurealkylester und/oder Propionsäurealkylester, und in eine Alkylmethacrylat-enthaltende Fraktion im Sumpf, die einen jeweiligen Gehalt an Methacrolein, Isobuttersäurealkylester und Propionsäurealkylester kleiner 0,1 Gew% aufweist.

Auch bevorzugt wird mindestens eine Alkymethacrylat-enthaltende Sumpf-Fraktion aus Destillationskolonne I oder aus Destillationskolonne II in einer Destillationskolonne III zur Abtrennung von schwersiedenden Bestandteilen geleitet. Optional kann diese von Schwersiedern gereinigte Fraktion danach in einer weiteren Destillationskolonne zur Abtrennung weiterer leichtsiedender Bestandteile weiter aufgereinigt werden (nicht Teil der Schemata im Anhang).

Optional kann die Destillation in Destillationskolonne I in Gegenwart eines zusätzlichen als Schleppmittel fungierenden Lösungsmittels durchgeführt werden.

In einer besonderen Ausführung der vorliegenden Erfindung wird die Kopf-Fraktion aus Destillationskolonne I und/oder die wässrige Phase aus dem Phasentrenner I in einen Reaktor IV geleitet. In diesem Reaktor IV erfolgt eine saure Hydrolyse.

Bevorzugt ist es auch, den Produktstrom aus Reaktor IV ganz oder teilweise einer Entsorgung zuzuführen und/oder in einen der vorgeschalteten Aufbereitungsschritte zu recyclieren.

Als besonders günstig hat es sich erwiesen, die Destillationskolonne I und die optionale Destillationskolonne II jeweils bei einem absoluten Druck zwischen 0,1 bar und 1 bar zu betreiben.

Eine besonders bevorzugte Variante des erfindungsgemäßen Verfahrens ist darüber hinaus dadurch gekennzeichnet, dass vor der Einleitung in Destillationskolonne I die extraktive Trennung in einer Extraktion I erfolgt. Bei dieser extraktiven Trennung wird eine Fraktion, die Wasser und Alkali- und/oder Erdalkalisalze enthält, abgetrennt. Eine Verfahrensoption stellt die Destillation der organischen Phase der Extraktion I in einer Destillationskolonne IV zur Abtrennung von Schwersiedern aus dem Roh-MMA dar. Die erzeugte schwersiedende Sumpffraktion kann danach in einer Destillationskolonne V nochmals destilliert werden. Das Destillat der Destillationskolonne V kann zur Minimierung von MMA-Verlusten in die Destillationskolonne IV zurückgeführt werden. Das Destillat der Destillationskolonne IV ist in dieser Verfahrensvariante der Zulauf der Destillationskolonne I.

Der Propionaldehydgehalt des Methacroleins aus Verfahrensschritt a liegt bevorzugt zwischen 100 Gew-ppm und 2 Gew%. Genauso bevorzugt liegt der Gehalt an Isobuttersäurealkylester im Alkylmethacrylat aus Verfahrensschritt b unter 2000 ppm.

Die Destillationsstufe I, bei der es sich um eine Leichtsiederkolonne handelt, kann auf verschiedene Art und Weise ausgelegt werden, um die benötigte Abtrennung der genannten Nebenprodukte Propionsäurealkylester und Isobuttersäurealkylester, sowie des verbleibenden Methacroleins zu erreichen. So hat es sich als praktikabel erwiesen, eine vielstufige Destillationskolonne mit Dekanter im Destillat einzusetzen. Die Destillationskolonne wird in der Regel bei einem Innendruck zwischen 100 mbar und 1 bar, bevorzugt zwischen 150 und 500 mbar und besonders bevorzugt zwischen 200 und 400 mbar betrieben. Dabei ergeben sich Sumpftemperaturen, die ungefähr bei 55 bis 100 °C liegen. Da bei höheren Temperaturen Nebenreaktionen, wie zum Beispiel eine Polymerisation auftreten können, ist es empfehlenswert den Druck derart einzustellen, dass die Sumpftemperatur unter 80 °C, bevorzugt unter 70 °C liegt. Die Kolonne ist bevorzugt derart ausgelegt und derart betrieben, dass die Kopftemperatur 7 bis 15 °C niedriger als die Sumpftemperatur.

Der Zulaufstrom besteht dabei überwiegend aus dem Alkylmethacrylat, bevorzugt MMA, dem Isobuttersäurealkylester, bevorzugt IBSME, dem Propionsäurealkylester, bevorzugt PRAME, Methacrolein, Methanol, Wasser und weiteren Leichtsiedern.

Vorzugsweise weist die Kolonne 30 bis 100, bevorzugt 45 bis 65 theoretische Stufen auf. Bei einer theoretischen Stufe handelt es sich um ein örtliches thermodynamisches Gleichgewicht innerhalb der Kolonne. Die Anzahl dieser kann auf zwei verschiedene Arten bzw. deren Kombinationen innerhalb einer Kolonne realisiert werden. Diese Modifikationen ergeben am Ende auch die benötigte Länge der Kolonne. Es können Siedeböden innerhalb der Kolonne vorliegen. Als zweite bzw. additive Methode können Füllkörper bzw. strukturierte Packungen in Bereichen der Kolonne eingefüllt sein. Der Zulauf in die Destillationskolonne I kann bevorzugt - vom Kolonnenboden aus gesehen - zwischen dem Sumpf und der Mitte der Kolonne erfolgen. Besonders bevorzugt erfolgt der Zulauf zwischen dem ersten Drittel und der Mitte der Kolonne.

In der Regel wird in der Destillationskolonne I bei relativ hohen Rückfluss-zu-Zulauf-Verhältnissen im Bereich von 0,5 bis 5,0, vorzugsweise von 1,0 bis 3,0, betrieben.

Das aufgereinigte MMA fällt im Sumpf der Kolonne an. Dabei sind die Betriebsbedingungen der Kolonne dem jeweiligen Aufbereitungskonzept anzupassen. Generell gilt, Leichtsieder, die in diesem MMA enthalten sind, können nicht durch Kolonnen zur Schwersiederabtrennung abgetrennt werden, sondern finden sich auch im Endprodukt wieder.

Nach Destillationskolonne I wird das Destillat unter optionaler Wasserzugabe einer Phasentrennung unterzogen und dabei werden ein wässriger und ein organischer Strom erzeugt. Der Phasentrenner kann bei einer Temperatur unter 50 °C betrieben werden. Bevorzugt liegt die Temperatur zwischen 4 und 30 °C, in der Regel zwischen 15 und 25 °C. Das Wasser-zu-Feed Verhältnis am Phasentrenner liegt in der Regel zwischen 0 (kein zusätzliches Wasser) und 0,5, bevorzugt zwischen 0,1 und 0,2. Der wässrige Strom enthält hauptsächlich H₂O, Methanol und einen bestimmten Teil der organischen Substanzen MMA, PRAME, IBSME, etc. Der wässrige Strom wird entweder als Abwasser behandelt oder kann einer optionalen Nebenproduktbehandlung, wie einer sauren Hydrolyse unterzogen werden. Bei der sauren Hydrolyse werden unter Zugabe einer in der Regel anorganischen Säure MMA, PRAME und IBSME wieder verseift. Dabei wird diese Verseifung bzgl. Temperatur, Säurekonzentration und Verweilzeit in Reaktor IV derart gesteuert, dass die Verseifung insgesamt nur unvollständig erfolgt. Da die Verseifungsreaktionen von PRAME und IBSME gegenüber MMA kinetisch bevorzugt sind, kann auf diese Art ein relevanter Anteil des MMA erhalten bleiben. Freigesetztes Methanol und verbleibendes MMA können alternativ nachfolgend, z.B. destillativ, ohne großen Aufwand isoliert werden. Optional kann das gesamte Produkt aus Reaktor IV in die Destillationskolonne VI, bei der es sich um die so genannte Methanol-Recovery Kolonne handelt, geleitet werden. Aufgrund des hohen Siedepunktes der freien Säuren können Isobuttersäure und Propionsäure dann einfach über den Sumpf der Destillationskolonne VI der Abwasseraufbereitung zugeführt werden.

Der organische Strom des Phasentrenner I wird vollständig der Kolonne zurückgeführt oder es wird optional ein Ausschleusstrom abgezogen. Optional lässt sich dieser Ausschleusstrom in einer weiteren Kolonne aufreinigen. Dabei wird MMA als Sumpfprodukt wiedergewonnen und die abzutrennenden Leichtsieder (IBSME, PRAME, MAL) als Destillat abgetrennt.

Es sei der Vollständigkeit halber festgestellt, dass alles in den Abschnitten zuvor über Methanol, MMA, PRAME und IBSME natürlich auch auf andere Alkohole und damit Alkylmethacrylate, Isobuttersäure- bzw. Propionsäurealkylester übertragbar ist.

Das erfindungsgemäße Verfahren erlaubt die Abtrennung von Isobuttersäuremethylester und gleichzeitig auch die Abtrennung von Methacrolein und Propionsäuremethylester in einer einzigen Destillationskolonne bei gleichzeitig MMA-Rückgewinnungsraten von über 99 Gew%.

### Beispiele

### Beispiel 1

In einer kontinuierlich betriebenen Anlage, dargestellt in Figur 1, wird der Destillatstrom (31) der Schwersieder-Kolonne (Destillationskolonne IV) (26) erzeugt, der in nachfolgenden Leichtsiederkolonne (Destillationskolonne I) (32) destillativ von den Leichtsiedern gereinigt wird. Durch die Operation kann ein Prozessstabilisatoren enthaltener MMA-Sumpfstrom erzeugt werden, der einen PRAME-Gehalt < 10 ppm und einen IBSME-Gehalt < 350 ppm aufweist.

Die Leichtsiederkolonne wird bei einem Betriebsdruck von 250 mbar absolut betrieben. Die Kolonne ist mit der Strukturpackung M750.Y der Firma Sulzer ausgestattet (Durchmesser 100 mm, Packungshöhe 12000 mm, Zulauf bei 6000 mm). Auf die Kondensatoren werden Prozessstabilisatoren gegeben. Der Prozessstabilisator ist in MMA gelöst und die Zugabe beträgt 330 g/h. Ein Phasentrenner, der auf eine Betriebstemperatur von 20 °C temperiert wird und über eine Wasserzugabe (34) verfügt, erzeugt eine wässrige Phase (36) und eine organische Phase. Diese organische Phase wird als Rücklauf in die Kolonne zurückgeführt und teilweise als Ausschleusstrom (35) aus dem Prozess ausgetragen.

Im vorliegenden Beispiel beträgt der Destillatstrom (31) der Schwersieder-Kolonne 11000 g/h und enthält 98,0 Gew% MMA, 1,0 Gew% H₂O, 0,2 Gew% MAL, 0,1 Gew% PRAME, 0,1 Gew% IBSME und 0,5 Gew% Rest, wobei es sich bei diesem Rest überwiegend um Methanol handelt. Ein Rückfluss-zu-Zulauf-Verhältnis von 1,1 ist eingestellt und der Ausschleusstrom ist auf 112 g/h fixiert. Die Rate der Wasserzugabe in den Phasentrenner beträgt 1285 g/h. Damit ergibt sich eine Kopftemperatur von 53 °C und eine Sumpftemperatur von 61 °C. Der wässrige Strom (36) des Phasentrenners beträgt 1483 g/h und enthält 1,2 Gew% MMA, 93,8 Gew% H₂O, 1 Gew% MAL, 0,3 Gew% PRAME, 0,1 Gew% IBSME und 3,6 Gew% Rest. Es ergibt sich ein Sumpfaustrag (40) von 10950 g/h mit der Zusammensetzung 99,9 Gew% MMA, 50 ppm PRAME, 350 ppm IBSME und 0,06 Gew% Prozessstabilisator. Im dargestellten Betrieb ist eine MMA-Rückhaltung von 99,04 % erzielt worden.

### Beispiel 2

Unter Verwendung der Kolonne (Betriebsdruck von 250 mbar absolut) und des Phasentrenner (Betriebstemperatur 20 °C) aus Beispiel 1 wird der Destillatstrom (31) der Schwersieder-Kolonne (11000 g/h, enthält 96,7 Gew% MMA, 1,8 Gew% H₂O, 0,1 Gew% MAL, 0,1 Gew% PRAME, 0,2 Gew% IBSME und 1,1 Gew% Rest) destilliert. Dabei ist das Rückfluss-zu-Zulauf-Verhältnis auf 2,2 eingestellt und der Ausschleusstrom auf 25 g/h fixiert. Die Wasserzugabe in den Phasentrenner beträgt 1530 g/h. Damit ergibt sich eine Kopftemperatur von 54 °C und eine Sumpftemperatur von 62 °C. Der wässrige Strom (36) des Phasentrenners beträgt 1858 g/h und enthält 1,6 Gew% MMA, 91,8 Gew% H₂O, 0,4 Gew% MAL, 0,4 Gew% PRAME, 0,5 Gew% IBSME und 4,9 Gew% Rest. Der Sumpfaustrag (40) beträgt 11035 g/h mit der Zusammensetzung 99,91 Gew% MMA, 35 ppm PRAME, 315 ppm IBSME und 0,06 Gew% Prozessstabilisator. Diese beschriebene Fahrweise erreicht eine MMA-Rückhaltung von 99,71 %.

### Beispiel 3

In der Kolonne (Betriebsdruck von 250 mbar absolut) und dem Phasentrenner (Betriebstemperatur 20 °C) aus Beispiel 1 wird der Destillatstrom (31) der Schwersieder-Kolonne (11000 g/h, enthält 96,7 Gew% MMA, 1,8 Gew% H₂O, 0,1 Gew% MAL, 0,1 Gew% PRAME, 0,2 Gew% IBSME und 1,1 Gew% Rest) destilliert. Dabei ist das Rückfluss-zu-Zulauf-Verhältnis auf 2,0 eingestellt und der Ausschleusstrom auf 25 g/h fixiert. Es erfolgt keine Wasserzugabe in den Phasentrenner. Damit ergibt sich eine Kopftemperatur von 50 °C und eine Sumpftemperatur von 62 °C. Der wässrige Strom (36) des Phasentrenners beträgt 335 g/h und enthält 5,5 Gew% MMA, 60,0 Gew% H₂O, 2,0 Gew% MAL, 1,5 Gew% PRAME, 2,0 Gew% IBSME und 29,0 Gew% Rest. Der Sumpfaustrag (40) beträgt 11000 g/h mit der Zusammensetzung 99,9 Gew% MMA, 55 ppm PRAME, 320 ppm IBSME und 0,06 Gew% Prozessstabilisator. Es wird eine MMA-Rückhaltung von 99,51 % erreicht.

### Beispiel 4

Die in den Beispielen 1 bis 3 angewandte Prozessverschaltung wird durch eine weitere Destillationskolonne II (44), die sogenannte Ausschleus-Kolonne, ergänzt (Figur 2). In diese Kolonne wird der Ausschleusstrom (35) als Zulauf gegeben und destillativ gereinigt. Das erzeugte Destillat (45) enthält dabei die leichtsiedenden Komponenten (MAL, PRAME und IBSME). Die Sumpf-Fraktion enthält MMA mit Prozessstabilisator und wird in die Leichtsiederkolonne zurückgeführt. Die Ausschleus-Kolonne wird bei einem Druck von 250 mbar absolut betrieben. Die Kolonne ist mit der Hochleistungslaborpackung DX der Firma Sulzer ausgestattet (Durchmesser 50 mm, Packungshöhe 2000 mm). Die Betriebsbedingungen der Leichtsiederkolonne und des Phasentrenner sind gleich dem Beispiel 1. Der Zulauf in die Leichtsiederkolonne beträgt 11000 g/h und enthält 96,7 Gew% MMA, 1,8 Gew% H₂O, 0,1 Gew% MAL, 0,1 Gew% PRAME, 0,2 Gew% IBSME und 1,1 Gew% Rest. Dabei ist das Rückfluss-zu-Zulauf-Verhältnis auf 1,0 eingestellt und die Wasserzugabe in den Phasentrenner beträgt 1530 g/h. Damit ergibt sich eine Kopftemperatur von 54 °C und eine Sumpftemperatur von 62 °C. Der Ausschleusstrom (35), der als Zulauf in die Ausschleus-Kolonne fungiert, ist auf 110 g/h fixiert. Der wässrige Strom (36) des Phasentrenners beträgt 1858 g/h und enthält 1,2 Gew% MMA, 93,8 Gew% H₂O, 1 Gew% MAL, 0,4 Gew% PRAME, 0,1 Gew% IBSME und 3,5 Gew% Rest. Die Kopftemperatur der Ausschleus-Kolonne ist 34 °C und eine Sumpftemperatur 61 °C. Es fällt ein Destillatstrom (45) der Ausschleus-Kolonne von 22 g/h enthaltend 2,1 Gew% MMA, 7,9 Gew% H₂O, 39,9 Gew% MAL, 26,6 Gew% PRAME, 19,5 Gew% IBSME und 4,0 Gew% Rest an. Der Sumpf (46) der Ausschleus-Kolonne wird vollständig auf die Leichtsiederkolonne zurückgeführt. Es ergibt sich ein Sumpf-Austrag (40) der Leichtsiederkolonne von 11038 g/h mit der Zusammensetzung 99,91 Gew% MMA, 5 ppm PRAME, 315 ppm IBSME und 0,06 Gew% Prozessstabilisator. Die MMA-Rückhaltung ist 99,73 %.

### Beispiel 5

Die in Beispiel 1 angewandte Prozessverschaltung wird um einen Rührkesselreaktor (Reaktor IV) (37) mit einem Reaktionsvolumen von 250 ml ergänzt (Figur 1). Der Rührer wird mit 500 U/min betrieben und die Betriebstemperatur ist 40 °C. In diesen Reaktor (37) wird die wässrige Phase (36) des Phasentrenner eingeleitet. Dieser Strom besteht aus 1,2 Gew% MMA, 93,8 Gew% H₂O, 1 Gew% MAL, 0,3 Gew% PRAME, 0,1 Gew% IBSME und 3,6 Gew% Rest und fällt mit einer Rate von 1483 g/h an. Zusätzlich wird 9,8 g/h 96 %-ige Schwefelsäure (38) in den Rührkesselreaktor (37) zugegeben. Mit den beschriebenen Strömen ergibt sich eine Verweilzeit von 10 min. Dabei wird ein PRAME-Umsatz von 44 %, IBSME-Umsatz von 48 % und ein MMA-Umsatz von 44 % bestimmt.

### Beispiel 6

Unter Verwendung der Kolonne (Betriebsdruck von 600 mbar absolut) und des Phasentrenner (Betriebstemperatur 20 °C) aus Beispiel 1 wird der Destillatstrom (31) der Schwersieder-Kolonne (11000 g/h, enthält 96,7 Gew% MMA, 1,8 Gew% H₂O, 0,1 Gew% MAL, 0,1 Gew% PRAME, 0,2 Gew% IBSME und 1,1 Gew% Rest) destilliert. Am Kopf der Kolonne wird Hexan als Schleppmittel aufgegeben. Der auszugleichende Hexan-Verlust ist 8,8 g/h. Dabei ist das Rückfluss-zu-Zulauf-Verhältnis auf 1,8 eingestellt und der Ausschleusstrom ist 73 g/h. Die Wasserzugabe in den Phasentrenner beträgt 1489 g/h. Damit ergibt sich eine Kopftemperatur von 54 °C und eine Sumpftemperatur von 84 °C. Im Kopf der Kolonne wird ein Hexan-Gehalt von 60,5 Gew% erreicht. Der wässrige Strom (36) des Phasentrenners beträgt 1816 g/h und enthält 0,4 Gew% MMA, 92,9 Gew% H₂O, 0,4 Gew% MAL, 0,4 Gew% PRAME, 0,4 Gew% IBSME und 5,5 Gew% Rest. Der Sumpfaustrag (40) beträgt 10930 g/h mit der Zusammensetzung 99,90 Gew% MMA, 35 ppm PRAME, 315 ppm IBSME und 0,07 Gew% Prozessstabilisator. Diese beschriebene Fahrweise erreicht eine MMA-Rückhaltung von 99,66 %.

### Bezugszeichenliste

**Figur 1****:** Gesamtfließbild der Herstellung von MMA ausgehend von Formalin und Propanal
**Figur 2****:** Gesamtfließbild der Herstellung von MMA ausgehend von Formalin und Propanal mit optionaler Ausschleus-Kolonne
   (1) Formalin-Zulauf in Reaktor I
   (2) Propanal-Zulauf in Reaktor I
   (3) Optionaler Stabilisator-Zulauf in Reaktor I
   (4) Reaktor I zur Methacroleinsynthese
   (5) Aufbereitung des Roh-Methacroleins
   (6) Methacrolein-Zulauf in Reaktor II
   (7) Reaktor II zur oxidativen Veresterung des Methacroleins
   (8) Alkohol-Zulauf (i.d.R. Methanol-Zulauf)
   (9) Sauerstoff- bzw. Luftzuleitung
   (10) Zulauf Base
   (11) Abgas Reaktor II
   (12) Reaktoraustrag Reaktor II
   (13) Destillationskolonne VII: Methacrolein-Recovery Kolonne
   (14) MAL Recycle
   (15) MAL-Acetal-Converter
   (16) Zulauf Säure (i.d.R. Schwefelsäure)
   (17) Produktstrom MAL-Acetal-Converter
   (18) Extraktion I
   (19) Wasser-Zulauf für Extraktion I
   (20) Wässrige Phase Extraktion I
   (21) Destillationskolonne VI: Methanol-Recovery Kolonne
   (22) Niedrigsiedende Fraktion, enthaltend Alkohol zur Rückführung in Reaktor II
   (23) Rückführung der Methanol-Recovery Kolonne
   (24) Sumpf-Fraktion, enthaltend Wasser, Säure und deren Alkalisalze, zur Entsorgung oder weiteren Aufarbeitung,
   (25) Organische Phase der Extraktion
   (26) Destillationskolonne IV: Schwersiederkolonne
   (27) Sumpf-Fraktion enthaltend MMA, Methacrylsäure und Schwersieder
   (28) Destillationskolonne V: MMA-Recovery Kolonne
   (29) Destillat enthaltend MMA
   (30) Sumpf-Fraktion enthaltend Methacrylsäure und Schwersieder
   (31) Destillat, enthaltend MMA und Leichtsieder
   (32) Destillationskolonne I: Leichtsiederkolonne
   (33) Optionaler Phasentrenner I
   (34) Optionale Wasserzugabe
   (35) Optionaler Ausschleusstrom
   (36) Optionale wässrige Phase des Phasentrenner I
   (37) Optionaler Reaktor IV saure Hydrolyse
   (38) Zulauf Säure (i.d.R. Schwefelsäure)
   (39) Produktstrom saure Hydrolyse, optional als Recyclierstrom
   (40) Sumpf-Fraktion der Leichtsiederkolonne
   (41) Destillationskolonne III: MMA Reinkolonne zur Endreinigung des MMA
   (42) Spezifikationsgerechtes MMA als Destillat der MMA Reinkolonne
   (43) Sumpf-Fraktion der MMA Reinkolonne optionale Rückführung zur Schwersiederkolonne
   (44) Destillationskolonne II: Ausschleus-Kolonne
   (45) Destillat enthaltend Leichtsieder, wie Isobuttersäuremethylester und Propionsäuremethylester
   (46) Sumpf-Fraktion der Ausschleus-Kolonne, enthaltend MMA
      (A) Abgas

Die Figuren stellen beispielhaft zwei verschiedene Ausführungen der vorliegenden Erfindungen dar. Dabei wirken die Darstellungen nicht einschränkend über den Inhalt der Ansprüche hinaus.

## Patentansprüche

1. Verfahren zur Herstellung von Alkylmethacrylaten, bei dem in einer ersten Reaktionsstufe in einem Reaktor I Methacrolein hergestellt und dieses in einer zweiten Reaktionsstufe in einem Reaktor II oxidativ mit einem Alkohol in Gegenwart eines sauerstoffhaltigen Gases verestert wird, unter Entstehung eines ungereinigten Alkylmethacrylatstroms, **dadurch gekennzeichnet, dass**
a. in Reaktor I ausgehend von Propionaldehyd und Formalin Methacrolein, enthaltend Propionaldehyd, erhalten wird,
b. in Reaktor II und einem optionalen Reaktor III das im Methacrolein enthaltene Propionaldehyd in Propionsäurealkylester umgewandelt wird und daneben optional Isobuttersäurealkylester entsteht,
c. nach Reaktor II oder Reaktor III der entstehende Alkylmethacrylat Roh-Strom, enthaltend Propionsäurealkylester und optional Isobuttersäurealkylester, in einem Aufarbeitungsverfahren zu reinem Alkylmethacrylat durch mehrere destillative Trennkolonnen und mindestens eine extraktive Trennung aufgearbeitet wird, aufweisend eine Destillationskolonne I, in der mittels Fraktionierung Alkylmethacrylat als Sumpf-Fraktion abgetrennt wird und die Nebenprodukt-Fraktion, enthaltend Methacrolein, sowie Isobuttersäurealkylester und Propionsäurealkylester, als Kopf-Fraktion erhalten wird.

2. Verfahren gemäß Anspruch 1, **dadurch gekennzeichnet, dass** aus Verfahrensschritt c. ein Alkylmethacrylat mit einem jeweiligen Gehalt an Propionsäurealkylester bzw. Isobuttersäurealkylester von unter 0,1 Gew% erhalten wird

3. Verfahren nach Anspruch 1 oder 2 **dadurch gekennzeichnet ist, dass** die Kopf-Fraktion aus Destillationskolonne I in einen Phasentrenner I geleitet wird und dort in eine wässrige und eine organische Fraktion getrennt wird.

4. Verfahren nach Anspruch 3 **dadurch gekennzeichnet ist, dass** in den Phasentrenner I zusätzlich Wasser geleitet wird, wobei es sich bei dem Wasser um Frischwasser und/oder um einen wasserhaltigen Recyclierungsstrom aus einem oder mehreren anderen Prozessschritten handeln kann.

5. Verfahren gemäß Anspruch 3 oder 4, **dadurch gekennzeichnet, dass** die organische Phase aus Phasentrenner I die Nebenprodukte Methacrolein, Isobuttersäurealkylester und Propionsäurealkylester enthält und teilweise oder vollständig in die Destillationskolonne I recycliert wird.

6. Verfahren gemäß mindesten einem der Ansprüche 1 bis 5, **dadurch gekennzeichnet, dass** der Kopf-Strom aus Destillationskolonne I und/oder die organische Phase aus dem Phasentrenner I ganz oder teilweise in eine Destillationskolonne II zur Fraktionierung geleitet wird, und dass in Destillationskolonne II eine Trennung in eine leichtsiedende Nebenprodukt-Fraktion, enthaltend Methacrolein, sowie Isobuttersäurealkylester und/oder Propionsäurealkylester, und in eine Alkylmethacrylat-enthaltende Fraktion im Sumpf, die einen jeweiligen Gehalt an Methacrolein, Isobuttersäurealkylester und Propionsäurealkylester kleiner 0,1 Gew% aufweist, trennt.

7. Verfahren gemäß mindesten einem der Ansprüche 1 bis 6, **dadurch gekennzeichnet, dass** mindestens eine Alkymethacrylat-enthaltende Sumpf-Fraktion aus Destillationskolonne I oder aus Destillationskolonne II in einer Destillationskolonne III zur Abtrennung von schwersiedenden Bestandteilen und danach in einer optionalen Destillationskolonne zur Abtrennung weiterer leichtsiedender Bestandteile weiter aufgereinigt wird.

8. Verfahren gemäß mindesten einem der Ansprüche 1 bis 7, **dadurch gekennzeichnet, dass** die Destillation in Destillationskolonne I in Gegenwart eines zusätzlichen als Schleppmittel fungierenden Lösungsmittels durchgeführt wird.

9. Verfahren gemäß mindesten einem der Ansprüche 1 bis 8, **dadurch gekennzeichnet, dass** die Kopf-Fraktion aus Destillationskolonne I oder die wässrige Phase aus dem Phasentrenner I in einen Reaktor IV geleitet wird, in dem eine saure Hydrolyse erfolgt.

10. Verfahren gemäß Anspruch 9, **dadurch gekennzeichnet, dass** der Produktstrom aus Reaktor IV ganz oder teilweise einer Entsorgung zugeführt wird und/oder in einen der vorgeschalteten Aufbereitungsschritte recycliert wird.

11. Verfahren gemäß mindesten einem der Ansprüche 1 bis 10, **dadurch gekennzeichnet, dass** Destillationskolonne I und die optionale Destillationskolonne II jeweils bei einem absoluten Druck zwischen 0,1 bar und 1 bar betrieben werden.

12. Verfahren gemäß mindesten einem der Ansprüche 1 bis 11, **dadurch gekennzeichnet, dass**, vor der Einleitung in Destillationskolonne I die extraktive Trennung in einer Extraktion I erfolgt, bei der eine Wasser und Alkali- und/oder Erdalkalisalze enthaltende Fraktion abgetrennt wird.

13. Verfahren gemäß mindesten einem der Ansprüche 1 bis 12, **dadurch gekennzeichnet, dass** der Propionaldehydgehalt des Methacroleins aus Verfahrensschritt a zwischen 100 ppm und 2 Gew% liegt.

14. Verfahren gemäß mindesten einem der Ansprüche 1 bis 13, **dadurch gekennzeichnet, dass** der Gehalt an Isobuttersäurealkylester im Alkylmethacrylat aus Verfahrensschritt c unter 2000 ppm liegt.

15. Verfahren gemäß mindesten einem der Ansprüche 1 bis 14, **dadurch gekennzeichnet, dass**, es sich bei dem Alkohol um Methanol, bei dem Alkylmethacrylat um MMA, bei dem Isobuttersäurealkylester um Isobuttersäuremethylester und bei dem Propionsäurealkylester um Propionsäuremethylester handelt.
